# EUROPEAN PATENT APPLICATION

(11) **EP 4 016 069 A1**
(43) Date of publication of application: **22.06.2022**
(21) Application number: 20215882.0
(22) Date of filing: 21.12.2020
(51) Int. Cl.: G01N 27/27, G01N 27/327, G01N 27/333, G01N 33/49, B01L 3/00

(54) **SENSOR DEVICE AND METHOD OF ITS USE**

(71) Applicant: F. Hoffmann-La Roche AG, 4070 Basel (CH); Roche Diagnostics GmbH, 68305 Mannheim (DE)
(72) Inventor: BOEHM, Christoph, 68305 Mannheim (DE); BURKHARDT, Wolfgang, 6343 Rotkreuz (CH); LUTZ, Sascha, 68305 Mannheim (DE); OCVIRK, Gregor, 68305 Mannheim (DE); RUF, Miriam, 68305 Mannheim (DE); KONTSCHIEDER, Heinz, 6343 Rotkreuz (CH)
(74) Representative: Curcio, Mario

(57) **Abstract**

The present disclosure refers to a sensor device (1, 1'), a method of operating the sensor device (1, 1') and an IVD analyzer (100) for receiving the sensor device (1, 1') for determining chemical and/or physical characteristics of a fluid. The sensor device (1, 1') comprises at least two fluidic conduits (2, 2', 3) for repeatedly receiving fluids, each fluidic conduit (2, 2', 3) comprising at least one sensory element (11A, 11B, 12A, 12B, 13, 14, 15) arranged such as to come in contact with a fluid in the respective fluidic conduit (2, 2', 3). In particular, the fluidic conduits (2, 2', 3) comprise different sensory elements, respectively, wherein the sensory elements are separated in the respective fluidic conduits according to compatibility or susceptibility to deterioration or operating temperature. Alternately, the fluidic conduits (2, 2', 3) comprise at least in part the same sensory elements, wherein the sensor device (1, 1') is operated in a primary operating mode and in response to a trigger event, switches to an extended operating mode.

## Description

### TECHNICAL FIELD

The present disclosure is related to a sensor device for an IVD analyzer that comprises sensors arranged in fluidic conduits. The present disclosure also relates to a method for operating the sensor device in the IVD analyzer.

### BACKGROUND

In-vitro diagnostic (IVD) analyzers such as blood gas and electrolyte analyzers can be used in critical care units, in the emergency room, in the hospital ward, in surgery units, in anesthesia, in outpatient clinics, in medical practices or during transport of patients. These are typically point of care settings, where there is a demand for short turn-around-times (TAT or STAT) of diagnostic results and/or where it is required to take multiple samples from a patient in short succession.

In blood gas and electrolyte testing, parameters are determined from a patient's sample, like the partial pressure of blood gases (*P*O₂, *P*CO₂), oxygen saturation (*S*O₂), the pH value, electrolyte concentrations (e.g. Na⁺, K⁺, Mg²⁺, Ca²⁺, Li⁺, Cl⁻), bicarbonate values (HCO₃⁻), the concentration of metabolites (e.g. glucose, lactate, urea, creatinine), values for hemoglobin and hemoglobin derivatives (e.g. tHb, O₂Hb, HHb, COHb, MetHb, SulfHb), bilirubin values, and hematocrit. These parameters allow a physician to obtain important information on heart function, lung function and kidney function of the patient.

Currently, a number of IVD analyzers is available on the market, which allow for measurements of these parameters with different degrees of automation. Generally, the parameters are determined by conductivity, electrochemical and/or optical measuring principles. In the latest generations of these IVD analyzers, the sensory elements required for these measuring principles are combined in a multi-use sensor device, to be inserted into an IVD analyzer. This allows for simultaneous determination of a plurality of parameters from one single sample in one single measurement. It is usually a goal to use the same sensor device for as many measurements as possible. However, when required, e.g. if one or more of the sensors reaches the end of their in-use time, the sensor device can be replaced with a new one.

The measurements take place in measuring chambers arranged inside the sensor device. These chambers can be designed as fluidic conduits, which are equipped with the respective conductivity, electrochemical, and/or optical sensory elements. In order to perform a measurement, a sample is introduced into the fluidic conduit so that it comes into contact with the sensory elements. After the measurement, the sample is removed from the fluidic conduit and replaced by other fluids, e.g. a stand-by solution, a cleaning solution, a quality control (QC) sample, a subsequent sample, a calibrator etc.

Measurements are usually performed on whole blood samples, ideally on arterial blood samples. The collection of arterial blood however is especially burdensome for patients. In certain patient groups, e.g. in neonates, capillary blood samples are drawn. This means that only limited volumes of sample material are available. It is therefore a general trend in blood gas and electrolyte testing to arrange as many sensory elements as possible into a sensor device and to minimize the size of the measuring chamber. This enables obtaining as many parameters as possible from one single sample, thus reducing the burden of multiple sample collections for the patient, and enables handling of small sample volumes.

However, implementing a plurality of sensory elements in a sensor device entails certain disadvantages. For example, US8262992B2 discloses a "modular sensor cassette", in which a plurality of sensory elements are arranged along a continuous fluidic conduit. Any fluid that is introduced into the fluidic conduit (e.g. sample, QC sample, calibrator, stand-by solution, cleaning solution) comes into contact with all available sensory elements. Consequently, compromises have to be made in the materials used for the sensory units and the composition of the fluids (where possible) in order to achieve compatibility between the fluids and the sensory elements. Also, it is known that some materials used for the sensory elements can leach between the sensory elements and thereby cause interferences.

Additionally, the in-use time of the sensor device is limited to the time when the first sensory element fails. Either the affected parameter is no longer available for the subsequent measurements, or the sensory element or the entire sensor device have to be replaced. Replacing a sensor device with a new one leads to an increased downtime of the IVD analyzer, including the time for replacement and the time during which the new sensor device requires initialization. Frequent sensor device replacement therefore represents a disadvantage in regard of economic efficiency and usability.

### GENERAL DESCRIPTION

It is against the above background that the embodiments of the present disclosure provide certain unobvious advantages and advancements over the prior art. In particular, a need was recognized for improvements of a sensor device for in-vitro diagnostic (IVD) analyzers.

Although the embodiments of the present disclosure are not limited to specific advantages or functionality, it is noted that the present disclosure allows for a sensor device for an IVD analyzer and a method for operating the sensor device in an IVD analyzer that extends the sensor device's in-use time, thereby reducing the number of sensor device replacements and consequently reducing the downtime of the IVD analyzer.

Another advantage of the sensor device and its method of use according to certain embodiments is that it enables interference-free and reliable determination of a plurality of parameters from one single sample with a small sample volume and without reducing the number of measurable parameters. This is achieved by separating the sensory elements in a particular manner in at least two fluidic conduits.

Another advantage of the sensor device and its method of use according to certain embodiments is that it enables the operation of sensory elements in optimized temperature ranges in order to extend their in-use time.

In particular, the present disclosure relates to a sensor device and a method for operating the sensor device in an IVD analyzer. The sensor device comprises at least two fluidic conduits for repeatedly receiving fluids, where each fluidic conduit comprises at least one sensory element arranged such as to come in contact with a fluid in the respective fluidic conduit. According to an embodiment, the at least two fluidic conduits comprise at least two primary fluidic conduits comprising different sensory elements, respectively, wherein the sensory elements are separated in the respective primary fluidic conduits according to any one or more criteria like their susceptibility to deterioration, their measuring principle, their operational conditions or their degree of interference. The method comprises enabling the at least two primary fluidic conduits to receive fluids and repeatedly providing fluids to the at least two primary fluidic conduits. According to another embodiment, the at least two fluidic conduits comprise at least one primary fluidic conduit and at least one secondary fluidic conduit, wherein the secondary fluidic conduit comprises at least in part the same sensory elements as the at least one primary fluidic conduit. The method comprises operating the sensor device in a primary operating mode, in which the at least one primary fluidic conduit is enabled to receive fluids and in which fluids are repeatedly provided to the at least one primary fluidic conduit. The method further comprises switching to an extended operating mode in response to a predetermined trigger event, in which the at least one secondary sample conduit is enabled to receive fluids and in which fluids are repeatedly provided to the at least one primary fluidic conduit and/or to the at least one secondary fluidic conduit.

The term "IVD analyzer" as used herein refers to an automated or semi-automated analytical apparatus configured to examine samples in vitro in order to provide information for screening, diagnosis and treatment monitoring purposes. The IVD analyzer is designed and adapted to the medical area of application, to the parameters to be determined and to corresponding laboratory workflows. For example, in a point-of-care testing environment, IVD analyzers can vary from handheld devices with low throughput, short turn-around time and limited number of measurable parameters to compact benchtop instruments with higher throughput and higher number of measureable parameters. Such IVD analyzers are designed to determine certain types of parameters, such as blood gases, electrolytes, metabolites, clinical chemistry analytes, immunochemistry analytes, coagulation parameters, hematology parameters, etc. Depending on the parameters of interest, a variety of different analytical methods and different detection technologies can be applied. For example, in the field of blood gas and electrolyte testing, electrochemical measuring principles and/or conductivity measuring principles and/or optical detection methods are used. An IVD analyzer comprises a plurality of functional units, each dedicated to a specific task and cooperating with each other in order to enable automated sample processing and analysis. Such functional units may be e.g. a fill port for receiving a sample, a pump, a valve, an analytical measurement unit, an optical detection unit, a hemolysis unit, a sample injection nozzle, a reagent storage, a temperature regulating unit, a controller, etc. One or more functional units may be integrated into a larger unit or module in order to simplify the operation of the IVD analyzer. An example of such a module is a fluid pack. It combines a fill port for receiving a sample, optical detection units, a fluid system, pumps, valves, pouches with system fluids, etc., and can be exchanged if required. Such an exchangeable module is also considered as part of the IVD analyzer in this disclosure, even if it is not a permanently installed part.

According to an embodiment, the IVD analyzer comprises a receptacle for receiving a sensor device, wherein the sensor device is in operative connection with the IVD analyzer. A "receptacle" as used herein refers to a functional unit of the IVD analyzer that is designed to hold a sensor device and allows the sensor device to come in operative connection with the IVD analyzer. The receptacle may therefore comprise connection elements that enable e.g. thermal, electrical, fluidic, optical or mechanical connection with the sensor device. The receptacle may be designed to allow multi-use sensor devices to be exchangeably inserted or it may be designed to have a sensor device permanently installed.

The IVD analyzer further comprises a controller. A "controller" as herein used is a programmable logic controller or processor running a computer-readable program provided with instructions to perform operations in accordance with an operation plan. The term can mean central processing units, microprocessors, microcontrollers, reduced instruction circuits (RISC), application specific integrated circuits (ASIC), logic circuits, and any other circuit or processor capable of executing the functions/methods described herein. Regardless of the type of processor, it is configured to execute one or more of the methods described herein. The controller may be integrated into the IVD analyzer, may be integrated into a unit, a sub-unit or a module of an IVD analyzer, or may be a separate logic entity in communication with the IVD analyzer or its units, sub-units or modules via a direct connection, wired or wirelessly, or indirectly over a communications network, wired or wirelessly, such as a wide area network, e.g. the Internet or a Health Care Provider's local area network or intranet, via a network interface device. In some embodiments, the controller might be integral with a data management unit, e.g. implemented on a computing device such as a desktop computer, a laptop, a smartphone, a tablet, PDA, etc., or it may be comprised by a server computer and/or be distributed/shared across/between a plurality of IVD analyzers. Moreover, the systems can include remote devices, servers and cloud-based elements that communicate via wires or wirelessly (e.g. infrared, cellular, Bluetooth^{®}), or a remote PC/server or a cloud-based system. The processor may be also configurable to control the IVD analyzer in a way that workflow(s) and workflow step(s) are conducted by the IVD analyzer.

In particular, according to an embodiment, the controller is configured to control the IVD analyzer to enable the at least two primary fluidic conduits of the sensor device to receive fluids and to repeatedly provide fluids to the at least two primary fluidic conduits. According to an embodiment, the controller may be configured in alternative or in addition to control the IVD analyzer to operate the sensor device in a primary operating mode, wherein the controller controls the IVD analyzer to enable the at least one primary fluidic conduit to receive fluids, and to provide fluids to the at least one primary fluidic conduit. In response to a predetermined trigger event, the controller controls the IVD analyzer to switch to an extended operating mode, wherein the controller controls the IVD analyzer to enable the at least one secondary sample conduit to receive fluids, and controls the IVD analyzer to provide fluids to the at least one primary fluidic conduit and/or to the at least one secondary fluidic conduit.

The term "sensor device" as used herein refers to a functional unit comprising more than one sensory element, where the sensory elements may be of the same type, e.g. based on the same functional principle and/or sensor design, or may be of a different type. The sensor device is typically designed as an exchangeable multi-use unit. However, the term "sensor device" as used herein may also refer to a functional unit that is permanently installed in an IVD analyzer. Typically, several hundred samples can be measured with one sensor device before it reaches the end of its in-use time. The sensory elements of the sensor device are typically applied to one or more substrates, where substrates are planar elements capable of carrying the necessary wiring for connecting the sensory elements with electrical contact elements. These contact elements are required to establish electrical connection with the IVD analyzer. A substrate has two opposite major surface areas. Sensory elements are typically applied to the same major surface area of the substrate. A sensor device may comprise a single substrate to which the sensory elements are applied. Alternatively, it may comprise a plurality of substrates, where each substrate carries at least one sensory element, and where the plurality of substrates are aligned side-by-side in a planar orientation so that the sensory elements face in the same direction. A substrate can be made of either an electrically non-conducting material, e.g. a polymer, ceramic, glass, or of a conducting material, e.g. metals like steel, aluminum, platinum, gold, or metal alloys. In the latter case, an insulation layer, e.g. a polymer layer or an epoxy resin, is applied between the sensor and the conductive substrate. A substrate may comprise or be in contact with a thermally conductive element, e.g. a metal or metal alloy element, or the substrate may be thermally conductive itself, e.g. a steel substrate. This allows for temperature exchange with a temperature regulating unit of the IVD analyzer, e.g. a heating coil or a Peltier element. The sensor device may comprise a housing to protect the sensors and wiring from external influences and to facilitate handling. The housing can be made of any non-conductive material to prevent influences on the electrochemical measurements.

The term "sensory element" is herein generically used to indicate a detector or a part of a detector configured to detect sample parameters by generating a correlated signal output that can be quantified and digitized. The sensory element can be selective or specific with respect to one sample parameter of interest or can be configured to detect and quantify a plurality of different sample parameters of interest. Therefore, the term "sensory element" can refer to a fully functional sensor, e.g. an electrochemical sensor, a conductivity sensor, an optical sensor, an enzyme-coupled sensor. The term can also refer to a part of a sensor (e.g. a working electrode, a reference electrode, a counter electrode) that in combination with one or more other sensory elements forms a fully functional sensor. The signal generated by the sensory element(s) may be a continuous signal over a period of time or it may refer to a single measurement point or a plurality of discrete measurement points over time.

The sensor device further comprises at least two fluidic conduits for repeatedly receiving fluids, wherein each fluidic conduit comprises at least one sensory element, i.e. the sensory elements are arranged on the substrate such that they are oriented towards the fluidic conduits in order to come in contact with an introduced fluid. The fluidic conduits can be formed either within the substrate or within a cover element covering the substrate or partially in both the substrate and the cover element. The cover element is a planar element that is applied onto the major surface area of the substrate that carries the sensory elements in order to protect the substrate and sensory elements from external influences. The cover element can be made of an inert, electrically non-conducting material, e.g. polymer, ceramic, or glass material. Alternatively, the housing can form the cover element. In another alternative, the fluidic conduits can be formed in a spacer element or partially in both the substrate and a spacer element. The spacer is a planar element that is arranged between and in parallel to the substrate and the cover element. The height of the spacer element is determined based on the required cross-sectional area of the fluidic conduit. Typically, the height of the spacer is comprised between 10 and 1000 um, e.g. between 50 and 600 um, or between 70 and 500 um. In order to prevent leakage of the introduced fluid, a sealing element may be applied between the substrate and the cover element or between the substrate and the spacer element to seal the at least two fluidic conduits. The sealing element may be a polymer with appropriate characteristics regarding viscosity and elasticity, e.g. an elastomer like a thermoplastic elastomer, rubber, silicone, latex. In an alternative, the spacer element itself can be designed to act as a sealing element. In another alternative, the cover element itself can be designed to act as a sealing element. When assembled, the substrate and cover element and alternatively the spacer element are attached to each other, which can be achieved e.g. with adhesives or welding (thermal or ultrasound) or with mechanical fastening methods, such as screws or bolts and the like.

The at least two fluidic conduits may be physically separated, i.e. each fluidic conduit has a separate fluidic inlet and fluidic outlet, or the at least two fluidic conduits may converge inside the sensor device and at least partially share a common fluidic pathway (e.g. a common fluidic inlet and/or a common fluidic outlet). Even though they are fluidically connected in the latter case, the fluidic conduits are referred to as separate fluidic conduits in this disclosure, because the common pathways are usually not critical for obtaining measurement results. With other words, the areas where sensory elements are present and measurements are conducted are spatially separated from each other.

The at least two fluidic conduits of the sensor device may comprise at least two primary fluidic conduits or they may comprise at least one primary fluidic conduit and at least one secondary fluidic conduit. The term "primary fluidic conduit" refers to the fluidic conduit that is primarily taken into operation. If a plurality of primary fluidic conduits are available, they are taken into operation at the same time. The process of putting a fluidic conduit into operation refers to any actions or procedures required to bring the fluidic conduit from an unused state to an in-use state, in which it can be used to perform sample measurements and consequently determine sample parameters of interest. For example, after a sensor device has been installed in the IVD analyzer, any available primary fluidic conduit is enabled to receive fluids. "Enabling to receive fluids" as used herein refers to adjusting the functional units of the IVD analyzer (e.g. pumps, valves, fluid system) in cooperation with the sensor device to allow fluids to be provided into the respective fluidic conduit (e.g., the valves in the IVD analyzer and/or the sensor device are switched to an appropriate position, the respective pumps in the IVD analyzer are in operating mode and any available seals or obstructions to the respective fluidic conduits are removed, etc.). A conditioning step may be performed by bringing the primary fluidic conduit(s) and the respective sensory element(s) in contact with a conditioning fluid. After completion of the conditioning step, one or more calibrations and optionally quality control (QC) measurements may be performed. Thereafter, the available primary fluidic conduit(s) is available for performing sample measurements, i.e. the primary fluidic conduit is in-use. If the sensor device comprises more than one primary fluidic conduit, sample measurements can be performed in the primary fluidic conduits simultaneously. Therefore, the sample is distributed into the plurality of available primary fluidic conduits. The same applies to other processes, e.g. QC samples or calibrators. Alternatively, sample measurements can be performed in a subset of the available primary fluidic conduits, depending on the available sensory elements and the requested test panel. In that case, the sample is directed into the respective primary fluidic conduit(s) required to perform the requested tests, while the sample is prevented from being directed into the other primary fluidic conduit(s). A stand-by solution may remain in the other primary fluidic conduit(s) to maintain its operability. If the sensor device comprises at least two primary fluidic conduits, the at least two primary fluidic conduits comprise different sensory elements, respectively, where the sensory elements are separated in the respective primary fluidic conduits according to any one or more criteria like their susceptibility to deterioration, their measuring principle, their operational conditions or their degree of interference.

The term "secondary fluidic conduit" as used herein refers to the type of fluidic conduit(s) that is taken into operation at a later time point than a primary fluidic conduit. That is, at the time point of taking the secondary fluidic conduit into operation, the primary fluidic conduit(s) is either in an in-use state or it has been in an in-use state. If a sensor device comprises two or more secondary fluidic conduits, the secondary fluidic conduits may be taken into operation at the same time or sequentially. For example, after a sensor device has been installed in the IVD analyzer, the sensor device is operated in a primary operating mode, in which the available primary fluidic conduit is enabled to receive fluids. Fluids are repeatedly provided to the primary fluidic conduit to perform e.g. a sample measurement, a calibration, a QC sample measurement or a conditioning step. During the primary operating mode, the secondary fluidic conduit is prevented from coming in contact with any fluid. The secondary fluidic conduit is thus not yet taken into operation and therefore not suitable for performing sample measurements, i.e. the secondary fluidic conduit is not in-use. However, in response to a predetermined trigger event, the method comprises switching to an extended operating mode, where the at least one secondary sample conduit is enabled to receive fluids. Fluids can then repeatedly be provided to the secondary fluidic conduit to perform e.g. a conditioning step, a calibration, a QC sample measurement or a sample measurement. The trigger event may be initiated automatically, e.g. when automatically detecting that at least one sensory element in the primary fluidic conduit is out of specification, or it may be initiated manually, e.g. by the operator of the IVD analyzer. If the sensor device comprises at least one primary fluidic conduit and at least one secondary fluidic conduit, the secondary fluidic conduit comprises at least in part the same sensory elements as the primary fluidic conduit. For example, the primary fluidic conduit may comprise sensory elements for determining e.g. pH, *P*O₂*, P*CO²*,* Na⁺, K⁺, Ca²⁺, Cl⁻, glucose and lactate. The secondary fluidic conduit may comprise sensory elements for determining e.g. pH, glucose and lactate. If it is detected that e.g. the sensory element for determining glucose is out of specification, the controller controls the IVD analyzer to switch to the extended operating mode. In this mode, the secondary fluidic conduit is enabled to receive fluids. This allows performing the measurements of glucose levels in subsequent samples by the sensory element for determining glucose in the secondary fluidic conduit, while the measurement signals from the sensory element for determining glucose in the primary fluidic conduit are omitted. At the same time, measurements for determining the other parameters are performed by the sensory elements in the primary fluidic conduit, since they still operate within specification. Thus, in the extended operating mode, fluids can be provided to both the primary fluidic conduit(s) and to the secondary fluidic conduit(s). For example, the fluid may be provided to both fluidic conduits at the same time or at least in part temporally overlapping or at different times. Alternatively, the extended operating mode may comprise providing fluids to the secondary fluidic conduit but not the primary fluidic conduit, e.g. when the secondary fluidic conduit comprises the same set of sensory elements as the primary fluidic conduit. In this case, the at least one secondary fluidic conduit may serve as substitute for the primary fluidic conduit. Providing substitute fluidic conduits allows for a longer in-use time of the sensor device. In another example, the extended operating mode may comprise providing two different fluids into both the primary and the secondary fluidic conduit, respectively. This allows for determining the same parameters in two different samples at the same time, thereby increasing sample throughput. In yet another example, a fluid is provided into both the primary and the secondary fluidic conduit, where comparison measurements for certain parameters may be performed in the secondary fluidic conduit that may be used to confirm a test result generated by one or more sensory elements in the primary fluidic conduit.

According to an embodiment, the extended operating mode comprises a conditioning step, where the conditioning step comprises providing a conditioning fluid to the at least one secondary fluidic conduit for conditioning of the at least one secondary fluidic conduit. The "conditioning step" as used herein refers to any procedure required to be performed to activate a fluidic conduit and its corresponding sensory elements and bring them to a state to generate accurate and reliable test results. Typically, a conditioning step is performed shortly after installation of the sensor device. In case of a sensor device with a primary fluidic conduit and a secondary fluidic conduit, where the secondary fluidic conduit is not put into operation immediately after installation, a first conditioning step may be performed for the primary fluidic conduit. A second conditioning step for the secondary fluidic conduit may be performed at a later time, e.g. in response to a trigger event. In order to perform the conditioning step in the extended operating mode, the secondary fluidic conduit is enabled to receive fluids. Then, the conditioning fluid is provided to the secondary fluidic conduit, optionally multiple times, where it remains for a predetermined amount of time in order to wet and activate the secondary fluidic conduit. The conditioning step may further comprise a connectivity check, in which thermal, electrical and fluidic connection between the secondary fluidic conduit and the IVD analyzer are checked. The conditioning step may further comprise a calibration.

According to an embodiment, the method for operating the sensor device comprises regulating the operating temperature of the at least one secondary fluidic conduit to be higher than the operating temperature of the at least one primary fluidic conduit while performing the conditioning step. In case of a sensor device with two or more secondary fluidic conduits that are taken into operation successively, the operating temperature may be regulated to be higher than the operating temperature of the primary fluidic conduit and of the in-use secondary fluidic conduit while performing the conditioning step for a further secondary fluidic conduit. The conditioning step may be performed faster and more effectively if the temperature is set to a higher value compared to the operating temperature for sample measurements. For example, for conditioning a sensory element for determining blood gases, the corresponding fluidic conduit may be heated to a temperature above 40° C, e.g. between 50° C and 55° C, while the operating temperature of the primary fluidic conduit is set to an operating temperature between 25° C and 40° C.

The term "fluid" as used herein is a generic term to indicate any type of liquid material that is processed in an IVD analyzer. It can refer to liquids that are sought to be analyzed, e.g. a sample, or to liquids that contain known levels of analytes and are used to confirm the IVD analyzer's operability, e.g. a QC sample or a calibrator or a reference solution. It can further refer to liquids that are used to bring or maintain the IVD analyzer in an operating mode, e.g. a stand-by solution/rinse solution or a conditioning fluid.

A "sample" refers to any biological material suspected of containing one or more analytes or having physical or chemical characteristics, the detection of which - qualitative and/or quantitative - may be associated to a medical condition. It can be derived from any biological source, such as a physiological fluid, including, blood, saliva, sputum, ocular lens fluid, cerebral spinal fluid (CSF), sweat, urine, milk, ascites fluid, mucous, synovial fluid, peritoneal fluid, pleural fluid, amniotic fluid, tissue, bone marrow, feces, cells or the like. The biological sample may be used directly as obtained from the source or following a pretreatment and/or sample preparation workflow to modify the character of the biological sample, such as preparing plasma from blood, diluting viscous fluids, lysis or the like. Methods of treatment can involve filtration, centrifugation, dilution, concentration, inactivation of interfering components, and the addition of reagents e.g. to enable carrying out one or more in-vitro diagnostic tests. The term "sample" is therefore not necessarily used to indicate the original sample but may also relate to a sample which has already been processed (pipetted, diluted, mixed with reagents, enriched, purified, amplified, etc.). According to an embodiment, the sample is a whole blood sample, where whole blood may be arterial, venous or capillary whole blood.

A "QC sample" refers to a fluid that mimics a sample and that contains known values of one or more QC substances. QC samples may be supplied in one or more levels, e.g. two or three levels that correspond to different concentration ranges of the QC substances. QC samples are typically measured in the same way and under the same conditions as samples, in order to check that a calibrated sensor is actually within the specifications or admissible range. A "QC substance" can be an analyte identical to an analyte of interest, the concentration of which is known, or that generates by reaction an analyte identical to an analyte of interest, the concentration of which is known, e.g. CO2 from bicarbonate, or it can be any other equivalent substance of known concentration, which mimics the analyte of interest or that can be otherwise correlated to a certain parameter of interest, e.g. a dye that behaves optically similar to hemoglobin or bilirubin.

A "calibrator" is a calibration solution that contains known values of one or more calibration materials used for calibration and that is measured under the same conditions as a sample. Typically, one or two calibrators are used for a one-point or two-point calibration, respectively, when the sensor responds linearly to analyte concentrations. Three or more calibrators may be used if the calibration curve is non-linear. In particular, also calibrators can be provided in different levels that correspond to different concentration ranges of the QC materials. A calibration material can be the same as a QC substance. For example, a calibration - typically a two-point calibration - may be performed to determine a signal slope. The "signal slope" can be used to determine the sensitivity of a sensor. If the signal slope reaches a predetermined lower slope limit or if it reaches a predetermined upper slope limit, the sensor's correct measurement performance cannot be further guaranteed and the sensor may be blocked for further measurements. The signal slope may further be compared to historical signal slope data. If the difference between the most recent signal slope and the previously determined signal slope values is too high, i.e. reaches a predetermined threshold, the calibration may be repeated, despite the most recent signal slope being inside the allowable range. In case the difference is too high for a predetermined number of times, the sensor may be blocked for further measurements.

For operation of ion-selective electrode sensors, a reference solution with a high content of e.g. KCl is required to obtain a liquid junction potential which is stable and substantially independent from the individual sample composition. The reference solution can be brought in contact e.g. with a reference electrode of a potentiometric sensor, which comprises a membrane specific for chloride. The constant KCl concentration allows the reference electrode to return a constant signal as compared to a signal returned by a working electrode of the potentiometric sensor, which is in direct contact with a sample of interest. The reference electrode may be placed in a fluidic conduit in close proximity to the corresponding working electrode or in a dedicated reference fluidic conduit. Reference electrode and reference fluidic conduit may be part of the sensor device, where a sensor device may comprise a plurality of reference electrodes and/or reference fluidic conduits. Alternatively, reference electrode and reference fluidic conduit may be part of the IVD analyzer, where the reference electrode and reference fluidic conduit are in operative connection with the corresponding working electrodes and fluidic conduits of the sensor device, respectively.

A "stand-by solution/rinse solution" is a solution that is used to rinse the sensory elements after sample measurement has been performed and is kept in contact with the sensory elements until it is replaced by another type of sample.

A "conditioning fluid" is a solution that is used to initialize a new, unwetted sensor. This conditioning step is usually performed when a new sensor or a new set of sensors is inserted into the IVD analyzer. It is a required procedure to ensure reliable operation of each sensor. The sensors are thereby contacted with conditioning fluid for a predetermined time. Alternatively, the conditioning fluid may be a whole blood sample. No sample measurements are performed during the conditioning step.

A "cleaning solution" is a solution that is used to rinse and clean the fluidic system and sensors after measurement of a sample. In order to enhance the cleaning solution's efficacy in removing traces of previous sample material or debris etc., it may comprise certain additives, e.g. detergents, sodium hypochlorite, biocide.

The above mentioned types of solutions may have different compositions or they may have partially or entirely the same compositions. Their naming therefore reflects their function. For example, a conditioning fluid may have the same composition as a stand-by solution. However, it is used for a different purpose and in a different manner.

The term "repeatedly receiving fluids" refers to the sensor device being designed as a multi-use unit. "Repeatedly" therefore means that - during the in-use time of a sensor device - a variety of different kinds of fluids can be directed sequentially through the fluidic conduits of the sensor device. For example, after a sample measurement has been completed, a stand-by solution/rinse solution is introduced into the fluidic conduit to rinse the sensor device, thereby moving the sample out of the sensor device through the fluidic outlet. The stand-by solution/rinse solution may then be removed from the sensor device when a new sample is introduced. In regular intervals, QC samples or calibrators can be introduced into the fluidic conduits.

The term "parameter" is herein used as general term indicating a constituent of a sample or a physical or chemical characteristic of a sample that can be determined and analyzed with suitable methods. For example, the term "parameter" can refer to an analyte, which is any substance or compound in a sample that an analytical method or test seeks to detect (e.g. chemical elements like ions, or molecules like peptides, proteins, RNA, DNA, fatty acids, carbohydrates and the like). It can also refer to physical or chemical characteristics of a sample, e.g. color, temperature, turbidity, viscosity, acidity, alkalinity, etc. In general, the information on presence, absence, concentration and/or the properties of a sample parameter may give an indication on the health status of a patient and thus may be used to derive a diagnosis, or it may be used to determine and regulate a therapeutic regimen. Further, a known analyte level may be used in e.g. a QC sample or calibrator in order to confirm that an IVD analyzer is still operating within specifications or admissible range. Examples of parameters of interest in the context of this disclosure are the partial pressure of gases, such as *P*O₂ and *P*CO₂*,* oxygen saturation (*S*O₂), blood electrolytes such as sodium (Na⁺), potassium (K⁺), magnesium (Mg²⁺), calcium (Ca²⁺), lithium (Li⁺), chloride (Cl⁻), protons (H⁺) in relation to pH, bicarbonate values (HCO₃⁻), and metabolites such as glucose, lactate, urea, creatinine. Other parameters of interest are hemoglobin, hemoglobin derivatives, such as deoxygenated hemoglobin, oxyhemoglobin, carboxyhemoglobin, methemoglobin, sulfhemoglobin, and bilirubin. An example of a physical parameter of interest is the hematocrit level.

According to an embodiment, the predetermined trigger event comprises detecting that the performance of at least one sensory element in the at least one primary fluidic conduit is out of specification. In such an event, the sensory element has aged or has deteriorated or has been damaged to an extent that reliable measurement results can no longer be guaranteed. In general, the trigger event is initiated when a predetermined threshold value is reached or exceeded (e.g. a predefined signal slope limit, a predefined number of successive out-of-range QC measurement results, a predefined number of days that the sensor device was in use), indicating that the sensory element is about to reach or has reached the end of its in-use time. For example, the trigger event may be initiated if the signal slope reaches a predetermined range limit or if the signal slope differs by a predetermined degree from historic signal slope data. Alternatively, the trigger event may be initiated if QC sample measurements are outside an admissible range for a predetermined number of times in a row, or it may be initiated if the sensory element fails to produce a stable electrical measuring signal. Alternatively, the trigger event may be initiated if the sensory element fails to generate measurement values throughout the measurement range, despite being (re-)calibrated. The threshold value may be set so that the trigger event is initiated with a certain lead time before the affected sensory element reaches the end of its in-use time. This enables a timely switch to the extended operating mode to initiate the secondary fluidic conduit for sample measurements and thus ensures a seamless transition from the defective sensory element in the primary fluidic conduit to the unused sensory element in the secondary fluidic conduit.

According to an embodiment, the method for operating a sensor device comprises regulating the operating temperature in each fluidic conduit separately. The sensor device therefore comprises a plurality of thermally conductive elements, wherein the substrates themselves may be thermally conductive or the thermally conductive elements are arranged adjacent to at least one of the substrates. "Operating temperature" as used herein refers to the temperature or temperature range inside the fluidic conduit that provides optimal conditions to perform a sample measurement for a parameter of interest. For example, when determining *P*O₂, operating temperature is set to approximately 37° C. Other sensor types may require other operating temperatures. For that reason, a controlled transfer of heat to or from the fluidic conduit may be established. Operating temperature may be regulated indirectly via the IVD analyzer that is equipped with the units required to transmit heat to or remove heat from the sensor device, e.g. by a heating coil, a Peltier element, a heat sink and the like. The sensor device is installed in the IVD analyzer so that the temperature regulating units of the IVD analyzer are in direct contact with a thermally conductive substrate or a thermally conductive element of the sensor device. The thermally conductive element may be attached to at least one substrate or it may be integrated into the housing of the sensor device. The surface of the thermally conductive element may cover a plurality of substrates, it may cover the entire major surface area of a single substrate, or it may partially cover a single substrate, e.g. confined to a certain region of a fluidic conduit. A substrate may be provided with a plurality of thermally conductive elements, if different regions in the fluidic conduit require different operating temperatures or if multiple fluidic conduits are available that require individual temperature-control. A thermally conductive element may be e.g. a metal element or a metal alloy element, or any other suitable material. Alternatively, the thermally conductive element may be designed to actively regulate temperature, e.g. if it is a Peltier element. In this case, the IVD analyzer may actuate the thermally conductive element by providing electrical power via corresponding electrical contact points on the sensor device.

According to an embodiment, the sensory elements of the sensor device comprise any of an electrochemical sensor or a conductivity sensor or an optical sensor. The term "electrochemical sensor" thereby comprises any type of sensor that converts a (bio)chemical reaction into an electrical signal. Different measuring principles may be applied depending on the parameter to be measured. According to a further embodiment, the sensory elements of the sensor device comprising an electrochemical sensor comprise any of an ion-selective electrode sensor or an enzyme-coupled sensor or a blood gas sensor. For example, electrolytes or ions can be determined by a potentiometric measurement principle. Potentiometric sensors measure the potential or voltage between two electrodes in a solution. A potentiometric sensor therefore usually comprises at least a working electrode (also referred to as measuring electrode) and a reference electrode. A membrane that is sensitive to a specific electrolyte or ion is arranged between the sample and the working electrode. The membrane usually has a complex composition comprising e.g. polymers, plasticizers, lipophilic salts and ionophores. Ionophores are a class of compounds that reversibly bind ions thereby increasing the membrane's permeability to a specific ion of interest and are selected according to the parameter to be measured by the sensor. The ion of interest interacts with the membrane, thereby generating a change of potential at the sample/membrane interface, which is detected by the working electrode. The reference electrode on the other hand is in direct contact with a reference solution, which itself is in contact with the sample (liquid junction), thus no sample induced change of potential is established. In this way, the reference sensor returns a constant signal independent of the ion concentration in the sample. The difference in voltage between the reference and working electrode is then used to calculate the concentration of the ion of interest in solution. In fact, the difference in voltage between the electrodes is proportional to the logarithm of the concentration of the ion of interest. Since these types of sensors are ion-selective, they are also referred to as ion-selective electrodes. They enable the measurement of many analytes, e.g. potassium (K⁺), sodium (Na⁺), calcium (Ca²⁺), chloride (Cl⁻), magnesium (Mg²⁺), lithium (Li⁺), etc., and/or the determination of chemical properties of a sample, e.g. by determining the sample's pH-value. The potentiometric measurement principle does however not only allow measurement of electrolytes or ions, but is also used to determine blood gas levels, e.g. *P*CO₂ (with a Severinghaus-type sensor), and/or metabolites, e.g. urea, ammonium. Apart from the working electrode and the reference electrode, a potentiometric sensor may comprise further correction electrodes for correcting potential background noise or interferences. Further, a potentiometric sensor may comprise an electrode complemented with a specific enzyme, i.e. the enzyme is applied to the electrolyte layer or electrolyte fluid between the membrane and the electrode. For example, Severinghaus-type sensors comprise carbon anhydrase, which in the presence of water catalyzes the hydration of carbon dioxide into hydrogen carbonate.

Another electrochemical measuring principle is the amperometric measurement principle. An amperometric sensor measures the flow of electric current between two electrodes, where the electric current is generated by oxidation/reduction reactions. An amperometric sensor therefore usually comprises at least a working electrode (also referred to as measuring electrode) and a counter electrode. In such a two-electrode arrangement, the counter electrode also functions as a reference electrode to act as a reference in measuring and stabilizing the potential of the working electrode. However, the reference electrode may also be implemented as a separate electrode besides a working and counter electrode. In the IVD field, different types of amperometric sensors are used. For example, a sensor for measuring partial pressure of oxygen (*P*O₂) may function according to the Clark measurement principle, in which oxygen diffuses through a membrane to a working electrode that is kept at a constant negative voltage in relation to the counter reference electrode. The oxygen is reduced at the working electrode, inducing an electric current between the working electrode and the counter reference electrode. The current can be measured and is proportional to the oxygen contained in the sample. Other types of amperometric sensors comprise enzyme-coupled electrodes, where the enzymes accelerate certain desired reactions. For example, certain glucose sensors based on glucose oxidase catalyze the conversion of glucose in the presence of water and oxygen to hydrogen peroxide and gluconic acid. The working electrode is held at a constant voltage relative to the reference electrode, which oxidizes the hydrogen peroxide, decomposing it to hydrogen ions, oxygen, and electrons. This induces a current that can be measured and is proportional to the glucose concentration in the sample. These sensors are also referred to as biosensors, due to the application of enzymes. Apart from measuring *P*O₂ and glucose, amperometric sensors can be also used to measure other metabolites, e.g. lactate, creatinine, creatine, etc.

The term "conductivity sensor" as used herein generally refers to a sensor that measures conductivity of the sample. Conductivity sensors are usually less complex than the above mentioned potentiometric or amperometric sensors. For example, they typically do not comprise an ion-specific membrane. A conductivity sensor typically comprises identically constructed electrodes and is used to measure the ability of a solution to conduct an electrical current. The electrical current increases in proportion to the number of ions (or charged particles) dissolved in the solution, their electrical charge and their mobility. A conductivity sensor may be used for detecting the presence or absence of a sample or other fluid in a fluidic conduit or it may be used for determining certain parameters, e.g. the hematocrit level, in a sample, or it may be used for detecting air bubbles or clots in a sample. It may further be used for calibration purposes.

The term "optical sensor" as used herein generally refers to a sensor that allows for optical detection of sample parameters and/or physical properties of a sample. Typical optical detection methods are e.g. photometry (e.g. light absorbance or scattering of light), fluorescence spectroscopy, turbidimetry based on scattering, fluorescence polarization. In order to enable optical detection, the cover element and/or the substrate can be made entirely of a transparent or translucent material (e.g. polypropylene, acrylic, polycarbonate, glass or the like) or they may contain transparent regions or recesses (optical measuring windows). For example, a metal substrate may have one or more recesses in the region of the fluidic conduits. This allows a light source installed in the IVD analyzer to illuminate the sample in the fluidic conduits. A photoreceptor detects the transmitted or emitted light from the sample, converting the electromagnetic energy into an electrical signal. Examples for photoreceptors are, photodiodes, including avalanche photodiodes, phototransistors, photoconductive detectors, linear sensor arrays, CCD detectors, CMOS optical detectors, including CMOS array detectors, photomultipliers, photomultiplier arrays, etc. Samples may be analyzed as such or after being diluted with another solution or after having been treated with reagents. Optical detection methods may be used to detect the result of a chemical or biological reaction or to monitor the progress of a chemical or biological reaction. The combination of light source and photoreceptor is regarded as sensor in the sense of this disclosure. An optical sensor may be used for determining certain parameters, e.g. O2, *S*O₂, tHb, O₂Hb, HHb, COHb, MetHb, SulfHb, and bilirubin.

According to an embodiment, the at least two primary fluidic conduits comprise different sensory elements, respectively, wherein the sensory elements are separated in the respective primary fluidic conduits e.g. according to their susceptibility to deterioration, to their measuring principle, to their operational conditions or their degree of interference.

According to another embodiment, the sensory elements that are the same in the at least one primary fluidic conduit and in the at least one secondary fluidic conduit are sensory elements with higher susceptibility to deterioration than the other sensory elements in the primary fluidic conduit.

According to yet another embodiment, the sensory elements with higher susceptibility to deterioration comprise any of an enzyme-coupled sensor and/or an ion-selective electrode sensor.

It is known that sensors can deteriorate over time. If a sensor is out of specification or admissible range repeatedly and cannot be brought back into specification through calibration, the sensor is no longer reliable and needs to be replaced. With other words, the sensor has reached the end of its in-use time. The rate of deterioration is highly dependent on the sensor's architecture, e.g. on the type of ionophores applied for ion selective electrodes. This naturally occurring deterioration can be predicted and taken into account when determining the sensor's expected in-use time. However, there are other factors that influence the rate of deterioration in an unpredictable way and may deteriorate a sensor at a faster rate than predicted, e.g. if a sensor has a high susceptibility to a certain compound in a liquid (e.g. in a reagent, a QC sample, a calibrator or a cleaning solution). In this disclosure, such sensors are referred to as having a "higher susceptibility to deterioration", as compared to sensors that are unaffected or minimally affected by said liquid. For example, an enzyme-coupled sensor may be highly susceptible towards a cleaning solution. Certain components of the cleaning solution, e.g. sodium hypochlorite, can interfere with and thereby denature the enzymes, thus progressively worsening the sensor's functionality in an unpredictable manner. The sensor's in-use time may become much shorter than anticipated, thereby also shortening the in-use time of the entire sensor device. Whereas the same cleaning solution may be ineffective or have a negligible effect towards sensors with a different architecture, e.g. a sensor for measuring sodium (Na⁺). Another example is a chloride sensor that may be susceptible towards certain QC substances, e.g. sulphorodamine B or tartrazin. Yet another example is a pH sensor that may be susceptible towards surfactants in a fluid. To increase the in-use time of the sensor device, it is of advantage to prevent the sensors with higher susceptibility to deterioration from coming in contact with said interfering liquid. It can therefore be advantageous to arrange them in a separate fluidic conduit, different from the fluidic conduit containing the sensors with lower susceptibility to deterioration, i.e. to separate them according to their susceptibility to deterioration. Valves and/or separated fluidic pathways may be implemented to prevent said interfering liquid from contacting the sensors with higher susceptibility to deterioration, while at the same time allowing supply of said liquid to the sensors with lower susceptibility to deterioration. Alternatively, the sensory elements with higher susceptibility to deterioration can be formed in duplicates in both a primary fluidic conduit and a secondary fluidic conduit. If the first sensory element in the primary fluidic conduit reaches the end of its in-use time, the secondary fluidic conduit can be enabled to receive fluids and perform sample measurements.

In another example, sensory elements can be separated between at least two primary fluidic conduits based on their measuring principle. In order to simplify manufacturing processes, it can be advantageous to group sensory elements with a similar structure and thus similar manufacturing procedures. Further, potential interference between the sensory elements can be prevented. Various measuring principles have previously already been described in this disclosure and will therefore not be discussed here.

In yet another example, sensory elements can be separated between at least two primary fluidic conduits based on their operational conditions. The term "operational conditions" as used herein generally refers to the conditions that ensure optimal operation of the sensory elements and the sensor device. For example, the term may refer to the temperature conditions under which sensory elements are ideally operated in order to improve the accuracy and reliability of measurement results. Alternatively, the term may refer to the frequency of test requests. In clinical practice, measurable parameters may be grouped into "panels" based on the frequency of test requests. For example, physicians more often request the determination of blood gas parameters together with electrolytes than the determination of metabolites, e.g. glucose or lactate. It may therefore be advantageous to physically separate sensory elements measuring parameters of different panels in a sensor device. Preventing sensors from unnecessarily coming in contact with sample material, thereby preventing unnecessary deterioration and thus extending their in-use time.

In yet another example, sensory elements can be separated between the at least two primary fluidic conduits based on their degree of interference. In particular, certain materials used for forming the sensory elements can leach between near-by sensory elements and thereby cause interferences. For example, the release of Ag ions from Ag/AgCl-electrodes may have an adverse effect on the stability of enzymes immobilized on the working electrode of amperometric sensors. In another example, leaching of enzymes from the working electrode of an amperometric sensor may have an adverse effect on the functionality of other amperometric working electrodes (e.g. a bovine-serum-albumin-working electrode may be contaminated by leaching of lactate oxidase and/or glucose oxidase from the lactate or glucose working electrode, respectively). In yet another example, leaching plasticizers and/or ionophores from ion-selective-electrode-membranes may have an adverse effect on the functionality of adjacently arranged potentiometric sensors. A physical separation of these sensory elements can therefore considered advantageous.

According to an embodiment, the sensor device comprises a reference conduit for receiving a reference solution. The reference conduit comprises at least one reference sensory element, the at least one reference sensory element being arranged to come in contact with the reference solution in the reference conduit. The at least one reference sensory element may further be in operative connection with at least a part of the sensory elements in the at least two fluidic conduits. In order to achieve a more stable electric potential at the reference sensory element of an electrochemical sensor, the reference sensory element is typically exposed to a reference solution with a known ion concentration, e.g. highly concentrated KCl solution, as opposed to performing a reference measurement with e.g. a sample. Therefore, the reference sensory element is formed in a separate reference conduit that enables providing a reference solution to the reference sensory element while the working electrode of the electrochemical sensor is in contact with the sample of interest. A plurality of working electrodes of different electrochemical sensors may thereby be in operative connection with the same reference sensory element or each working electrode may be in operative connection with a corresponding separate reference sensory element. A sensor device may comprise a plurality of reference conduits if required. As an alternative to arranging the reference conduit in the sensor device, it can also be integrated into a functional unit of the IVD analyzer.

According to an embodiment, the sensor device comprises at least one common fluidic inlet for the at least two fluidic conduits and a switchable valve for directing fluids into any of the at least two fluidic conduits separately or simultaneously. The fluidic inlet is further fluidically connected to the fluid system of the IVD analyzer in order to enable the transfer of fluids from the IVD analyzer into the sensor device. The switchable valve is in operative connection with the IVD analyzer, where the operative connection can be e.g. mechanical, conductive, magnetic, or any other suitable mechanism. Hence, the controller controls the IVD analyzer to switch the switchable valve into the respective position to direct an available sample into the fluidic conduit(s) of choice. In an example, instead of valves, other physical obstructions may be installed in the fluidic conduits, e.g. gas or liquid pouches, movable flaps, shutter-like constructions, and the like. In another example, the switchable valve or other physical obstructions may be installed in the fluid system of the IVD analyzer. In that case, the sensor device may comprise a plurality of fluidic inlets, where each of the at least two fluidic conduits of the sensor device may be fluidically connected to a separate fluidic inlet, respectively.

According to an embodiment, the sensor device comprises a fluidic outlet in fluidic connection with each of the at least two fluidic conduits and with the reference conduit. The fluidic outlet is further fluidically connected to the fluid system of the IVD analyzer in order to enable the transfer of fluids out of the sensor device back to the IVD analyzer, where they can be wasted. Alternatively, each of the at least two fluidic conduits and the reference conduit may be fluidically connected to a separate fluidic outlet, respectively.

### BRIEF DESCRIPTION OF THE DRAWINGS

- FIG. 1: shows a flow diagram of a method for operating a sensor device as illustrated in FIG. 3;
- FIG. 2: shows a flow diagram of a method for operating a sensor device as illustrated in FIG. 4;
- FIG. 3: is a schematic illustration of a sensor device according to an embodiment comprising two primary fluidic conduits;
- FIG. 4: is a schematic illustration of a sensor device according to a further embodiment comprising a primary fluidic conduit and a secondary fluidic conduit;
- FIG. 5: is a schematic illustration of a sensor device according to an embodiment comprising a plurality of primary fluidic conduits and a secondary fluidic conduit;
- FIG. 6: is a schematic illustration of an IVD analyzer.

Skilled artisans appreciate that elements in the figures are illustrated schematically for simplicity and clarity and have not necessarily been drawn to scale. For example, the dimensions of some of the elements in the figures may be exaggerated relative to other elements to help improve understanding of the embodiments of the present disclosure. Also, parts have been omitted that do not contribute to the teaching of this disclosure.

### DETAILED DESCRIPTION

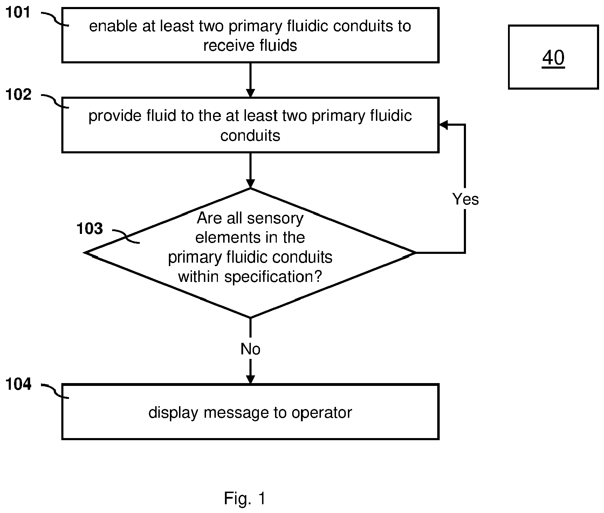

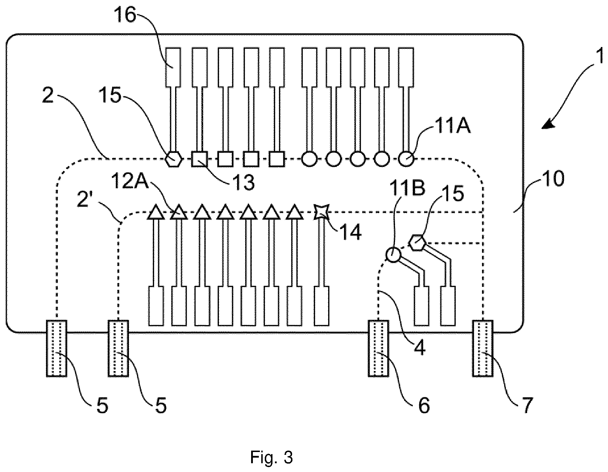

**FIG. 1** shows a flow diagram illustrating a method for operating a sensor device as referenced in **FIG. 3****,** wherein the sensor device comprises at least two primary fluidic conduits comprising different sensory elements, respectively. A controller 40 is configured to control the in-vitro diagnostic (IVD) analyzer to perform the method according to **FIG. 1****,** wherein the method comprises enabling the at least two primary fluidic conduits of the sensor device to receive fluids 101. The enabling step 101 can comprise a plurality of operations to set up the functional units of the IVD analyzer in cooperation with the sensor device in order to allow fluids to be provided from the IVD analyzer into the at least two primary fluidic conduits of the sensor device. For example, it may involve switching the valves in the IVD analyzer and/or sensor device to appropriate positions. It may further comprise initiating the respective pumps in the IVD analyzer or removing any available seals or obstructions that prevent fluids from being provided to the at least two primary fluidic conduits. It may further comprise initiating the fluid system of the IVD analyzer. In a further step 102, a respective fluid is provided to the at least two primary fluidic conduits, in order to perform an operation workflow, like a conditioning step, a calibration, a quality control (QC) sample measurement, a sample measurement, a cleaning procedure, etc. The sensory elements in the at least two primary fluidic conduits are constantly monitored to ensure their correct and reliable operation 103. As long as the sensory elements in the at least two primary fluidic conduits are within specification, the sensor device remains in-use and the two primary fluidic conduits can repeatedly be provided with respective fluids 102. If at least one of the sensory elements in the at least two primary fluidic conduits of the sensor device becomes defective, i.e. if it falls out of specification, the sensor device can no longer generate reliable measurement results. Therefore, a corresponding message is displayed 104 to the operator, indicating that the sensor device should be checked or replaced.

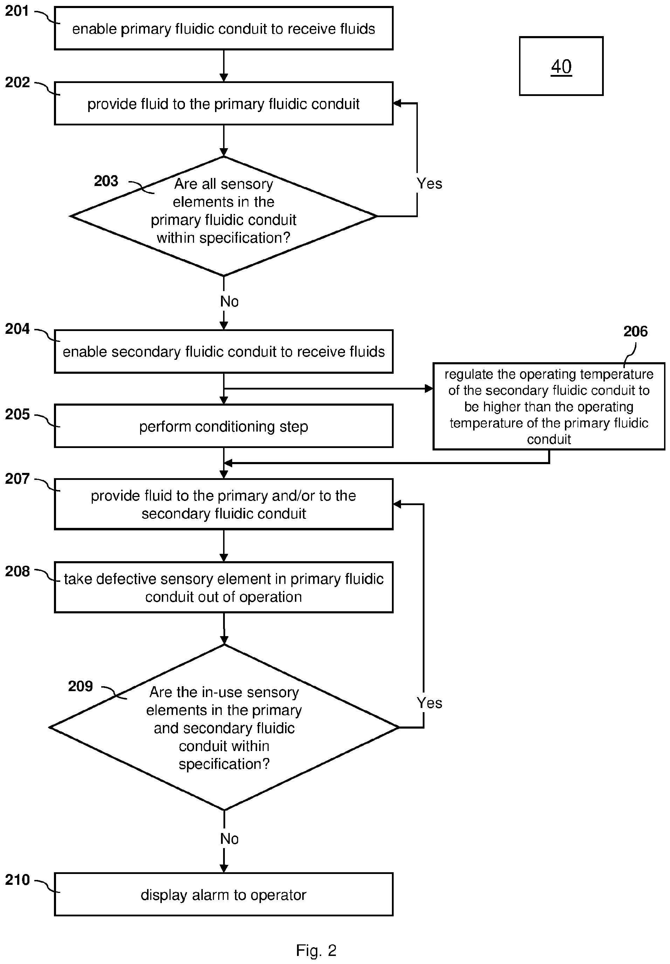

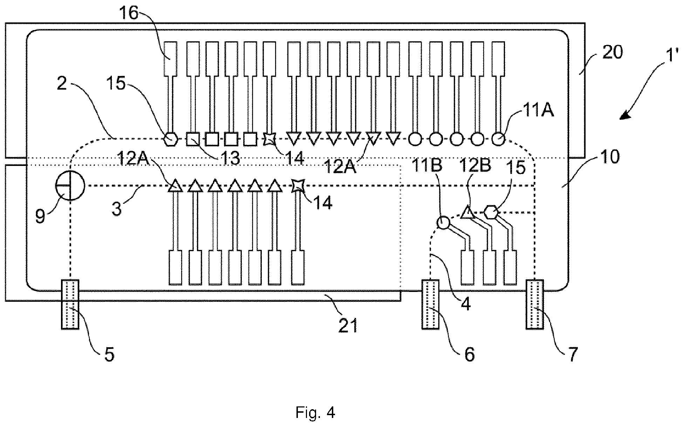

The flow diagram depicted in **FIG. 2** illustrates an embodiment of an alternative method for operating a sensor device as referenced in **FIG. 4****,** wherein the sensor device comprises at least one primary fluidic conduit and at least one secondary fluidic conduit, the secondary fluidic conduit comprising at least in part the same sensory elements as the at least one primary fluidic conduit. A controller 40 is configured to control the IVD analyzer to perform the method according to **FIG. 2****,** where the method comprises operating the sensor device in a primary operating mode. In the primary operating mode, the at least one primary fluidic conduit is enabled to receive fluids 201. As discussed in connection with **FIG. 1** above, the enablement of the primary fluidic conduit 201 may comprise a plurality of operations to set up the functional units of the IVD analyzer in cooperation with the sensor device. In a further step 202, a respective fluid is provided to the at least one primary fluidic conduit in order to perform a certain operation workflow (e.g. a conditioning step, a calibration, or a QC sample measurement, a sample measurement or a cleaning procedure). The sensory elements in the at least one primary fluidic conduit are monitored to ensure their correct and reliable operation 203. Monitoring can be performed continuously or in regular time intervals. As long as the sensory elements in the at least one primary fluidic conduit are within specification, the sensor device is operated in the primary operating mode and the at least one primary fluidic conduit can repeatedly be provided with respective fluids 202.

In response to a predetermined trigger event, the controller 40 controls the IVD analyzer to switch operation of the sensor device to an extended operating mode, wherein the trigger event comprises detecting that the performance of at least one sensory element in the at least one primary fluidic conduit is out of specification. The extended operating mode comprises enabling the at least one secondary fluidic conduit to receive fluids 204. The extended operating mode further comprises a conditioning step 205. The conditioning step comprises providing a conditioning fluid to the at least one secondary fluidic conduit where it may remain for a predetermined amount of time in order to wet and activate the secondary fluidic conduit. In the embodiment shown in **FIG. 2****,** the operating temperature of the secondary fluidic conduit can be regulated to be different than the operating temperature of the primary fluidic conduit 206 while the conditioning step 205 is performed. For example, for conditioning a sensory element for determining blood gases, the secondary fluidic conduit may be heated to a temperature of about 50° C to 55° C, while the operating temperature of the primary fluidic conduit is set to a temperature between 25° C and 40° C. This may accelerate conditioning of the secondary fluidic conduit. The conditioning step 205 may further comprise a connectivity check and/or a calibration. The enablement 204 of the secondary fluidic conduit to receive fluids and the conditioning step 205 can occur while the primary fluidic conduit is still in operation, e.g. performing sample measurements.

After completion of the conditioning step 205, the secondary fluidic conduit is in-use. In the subsequent operation workflow, the controller 40 controls the IVD analyzer to provide a fluid to the at least one primary fluidic conduit and/or to the at least one secondary fluidic conduit 207. The defective sensory element in the primary fluidic conduit is taken out of operation 208. Alternatively, the entire set of sensory elements that is the same in both the primary fluidic conduit and the secondary fluidic conduit may be taken out of operation in the primary fluidic conduit. "Take out of operation" refers to a software controlled process rather than a physical removal of the defective sensory element or set of sensory elements. In particular, in any of the subsequently performed operation workflows (e.g. sample measurements, QC sample measurements, calibrations), the defective sensory element or set of sensory elements in the primary fluidic conduit may no longer be read out or its measurement signal(s) may be disregarded. The test parameter will however be measured by a sensory element of the same type in the secondary fluidic conduit. The sensory elements still in-use in the primary fluidic conduit and/or the secondary fluidic conduit are monitored to ensure their correct and reliable operation 209. As long as the sensory elements still in-use in the primary fluidic conduit and the secondary fluidic conduit are within specification, the sensor device is continuously operated in the extended operating mode and the at least one primary fluidic conduit and the at least one secondary fluidic conduit can repeatedly be provided with respective fluids 207. If at least one of the sensory elements still in-use in the at least one primary fluidic conduit and the at least one secondary fluidic conduit falls out of specification, the sensor device can no longer generate reliable measurement results. Therefore, an alarm is triggered and the operator is informed to check the sensor device or replace it 210.

With reference to **FIG. 3****,** an embodiment of a sensor device 1 comprising two primary fluidic conduits 2, 2' is schematically illustrated. The primary fluidic conduits 2, 2' may be formed either within the substrate 10 or within a cover element (not shown) covering the substrate 10 or within a spacer element (not shown) positioned between the substrate 10 and the cover element. Alternatively, the primary fluidic conduits 2, 2' may be formed partially in both the substrate 10 and the cover element or the substrate 10 and the spacer element. The primary fluidic conduits 2, 2' are indicated by dashed lines. The sensor device 1 may further comprise a sealing element (not shown) applied between the substrate 10 and the cover element or the spacer element, respectively. The sealing element may be made of an inert material with appropriate characteristics regarding viscosity and elasticity (e.g. an elastomer like a thermoplastic elastomer, rubber, silicone, latex etc.), in order to seal the primary fluidic conduits 2, 2' and prevent leakage. The substrate 10 may be made of an electrically non-conductive material, e.g. a polymer, a ceramic, a glass material, or an electrically conductive material, e.g. a metal or a metal alloy. The substrate 10 may be a steel substrate. An insulation layer (not shown) may be formed between the sensory elements 11A, 11B, 12A, 13, 14, 15 and the steel substrate 10. In another example, the substrate 10 may be a transparent polymer substrate to enable optical detection. Each of the two primary fluidic conduits 2, 2' has a separate fluidic inlet 5, where the fluidic inlets 5 represent the fluidic contact elements to the IVD analyzer.

When the sensor device 1 is positioned in the IVD analyzer, the steel substrate 10, which is thermally conductive, comes in operative connection with a temperature regulating unit of the IVD analyzer, e.g. a heating coil, a Peltier element, a heat sink (not shown). The IVD analyzer controls the temperature regulating unit according to a predetermined protocol to transfer heat to or from the primary fluidic conduits 2, 2' via the thermally conductive substrate 10. Thereby, the operating temperature in the fluidic conduits 2, 2' can be regulated.

A plurality of sensory elements 11A, 11B, 12A, 13, 14, 15 are formed on the substrate 10 facing the primary fluidic conduits 2, 2'. In the embodiment depicted in **FIG. 3****,** the sensor device 1 comprises electrochemical sensors 11A, 11B, 12A, 13, 14 and conductivity sensors 15. The group of electrochemical sensors thereby comprises ion-selective electrode sensors 11A, 11B (indicated as circles), enzyme-coupled sensors 12A (indicated as triangles), blood gas sensors 13 (indicated as squares), and a pH sensor 14 (indicated as square with vaulted sides). Ion-selective electrode sensors 11A, 11B can be used to detect analytes such as Na⁺, Ca²⁺, K⁺, and Cl⁻, etc. According to the embodiment illustrated in **FIG. 3****,** the ion-selective electrode sensors 11A, 11B comprise working electrodes 11A, formed in a first primary fluidic conduit 2, and a reference electrode 11B formed in a reference conduit 4, wherein the working electrodes 11A and the reference electrode 11B are in operative connection with each other. The enzyme-coupled sensors 12A can be used to detect metabolites in a sample, e.g. glucose or lactate. The blood gas sensors 13 can be used to determine e.g. *P*O₂*, P*CO₂, and the pH sensor 14 can be used to determine the pH value of a sample. Further, conductivity sensors 15 (indicated as hexagons) are formed on the substrate 10 to measure the ability of a fluid to conduct an electrical current. This can be used to determine e.g. the hematocrit level in a sample, to differentiate between different types of fluids present in the primary fluidic conduits 2, 2' or reference conduit 4, or to detect bubbles or clots in the introduced fluid.

In an example, the substrate 10, the cover element and/or the spacer element and/or the sealing element may at least partially be made of a transparent polymeric material to enable optical detection. An optical detection unit, comprising e.g. a light source and a photoreceptor, may be installed in the IVD analyzer (not shown). Once the sensor device 1 is positioned in the IVD analyzer, the optical detection unit is in alignment with one or more of the fluidic conduits 2, 2'. The light source (not shown) may be positioned above the substrate 10 to emit light that is directed through a fluid in a fluidic conduit 2, 2' and detected by a photoreceptor (not shown) positioned below the substrate 10. The substrate 10 and/or the cover element and/or the spacer element and/or the sealing element may therefore comprise an aperture to allow the transmitted light to reach the photoreceptor. The aperture can be a recess that is filled with a transparent material, e.g. polymer or glass. Alternatively, both the light source and the photoreceptor may be positioned on the same side of the sensor device 1, e.g. above the substrate 10. In an example, the optical detection method used to determine oxygen levels in a given sample may be based on fluorescence quenching. Thereby, a fluorescence signal inversely proportional to oxygen levels in the sample is detected by the photoreceptor and converted into an electric signal. The samples may have to be preprocessed, e.g. they may have to be treated with a reagent prior to the measurement.

In order to perform operation workflows (e.g. sample measurement, QC sample measurement, calibration, rinsing, cleaning, conditioning), a respective fluid can be provided into both primary fluidic conduits 2, 2' or into either one of the primary fluidic conduits 2, 2' separately. For example, a biological sample can be introduced into the primary fluidic conduits 2, 2' via respective fluidic inlets 5, where it comes in contact with the sensory elements 11A, 12A, 13, 14, 15. A sample measurement can then be performed in order to detect sample parameters of interest.

The sensory elements 11A, 11B, 12A, 13, 14, 15 can be formed in either the first primary fluidic conduit 2 or the second primary fluidic conduit 2'. The rationale for arranging a sensory element in one of the two primary fluidic conduits 2, 2' can be based on, for example, its susceptibility to deterioration, its measuring principle, its operational conditions or its degree of interference with other sensory elements or other criteria. In the embodiment depicted in **FIG. 3****,** the sensory elements 11A, 11B, 12A, 13, 14, 15 are distributed in either the first primary fluidic conduit 2 or the second primary fluidic conduit 2' based on their susceptibility to deterioration. For example, the sensory elements comprising working electrodes of ion-selective electrode sensors 11A, blood gas sensors 13 and a conductivity sensor 15 are formed in a first primary fluidic conduit 2. The sensory elements comprising enzyme-coupled sensors 12A and a pH sensor 14 are formed in a second primary fluidic conduit 2', wherein these sensors have a higher susceptibility to deterioration as compared to the sensory elements 11A, 13, 15 in the first primary fluidic conduit 2. With the setup as illustrated in **FIG. 3****,** it can be prevented that the sensory elements with higher susceptibility to deterioration 12A, 14 come in contact with potentially deteriorating fluids (e.g. reagent, QC sample, cleaning solution). The IVD analyzer controls the respective fluids to be directed into the first primary fluidic conduit 2 but not into the second primary fluidic conduit 2'.

In the embodiment depicted in **FIG. 3****,** the reference conduit 4 is designated for receiving a reference solution in order to conduct reference measurements. The reference conduit 4 has a separate fluidic inlet 6 through which the reference solution is provided into the sensor device 1 from a reservoir in the IVD analyzer (not shown). The reference electrode of an ion-selective electrode sensor 11B and a conductivity sensor 15 are formed along the reference conduit 4. The primary fluidic conduits 2, 2' and the reference conduit 4 converge and lead into a common fluidic outlet 7, through which the fluid is transferred out of the sensor device 1 and back into the fluid system of the IVD analyzer, where it can be wasted (not shown). In order to establish fluidic connection with corresponding counterparts in the IVD analyzer, the fluidic inlets 5, 6 and the fluidic outlet 7 may protrude from the substrate 10, which is indicated in **FIG. 3****,** e.g. in a tubular shape, thereby forming plug-in connectors. The protrusions may be formed in a direction in parallel to the flow direction of the fluidic conduit. Alternatively, the fluidic connection between the sensor device 1 and the IVD analyzer may be established at any angle between 1° and 90°. The counterparts in the IVD analyzer would then be designed as mating connectors to the plug-in connectors. The plug-in connectors may be formed by the substrate 10, they may be formed by a housing (not shown) that encloses the sensor device 1, they may be formed by a cover element (not shown) covering the substrate 10 or they may partially be formed by any of the aforementioned elements. Alternatively, the plug-in connectors may be formed in the IVD analyzer and the mating connectors may be formed by the sensor device.

The electrical contact elements 16 are used to establish electrical connection between the sensory elements of the sensor device 1 and the IVD analyzer. In the embodiment as shown in **FIG. 3****,** the sensory elements 11A, 11B, 12A, 13, 14, 15 are connected to the corresponding electrical contact elements 16 via electrically conducting pathways printed onto the substrate 10, e.g. platinum pathways.

When assembled during manufacturing, the substrate 10 may be attached to a cover element (not shown) and/or a spacer element (not shown) and optionally a sealing element (not shown). This may be achieved, for example, with adhesive or by welding, or with mechanical fastening methods, such as screws or bolts and the like. The sensor device 1 further comprises a housing (not shown), typically made of an electrically non-conducting material, e.g. a polymer, to protect the sensory elements and wiring from external influences and to facilitate handling. In an example, the sensor device 1 including its housing can be exchangeably insertable into a corresponding receptacle of the IVD analyzer (not shown), where the analytical measurements are then performed.

**FIG. 4** schematically illustrates another example of a sensor device 1' according to further embodiments of the present disclosure. The sensor device 1' comprises a primary fluidic conduit 2 and a secondary fluidic conduit 3, wherein the secondary fluidic conduit 3 comprises at least in part the same sensory elements as the primary fluidic conduit 2. The primary fluidic conduit 2 and the secondary fluidic conduit 3 are indicated by dashed lines. The sensor device 1' further comprises a substrate 10, on to which the sensory elements 11A, 11B, 12A, 12B, 13, 14, 15 are formed.

The sensor device 1' further comprises a plurality of thermally conductive elements 20, 21 for regulating the operating temperature in each fluidic conduit separately. In the embodiment depicted in **FIG. 4****,** two thermally conductive elements 20, 21 are arranged adjacent to the substrate 10. **FIG. 4** shows a top view of substrate 10, with the thermally conductive elements 20, 21 arranged below the substrate 10. The thermally conductive elements 20, 21 are therefore indicated by dotted lines where they are not directly visible. The thermally conductive elements 20, 21 are made of a thermally conductive material, e.g. metal or a metal alloy. Each of the thermally conductive elements 20, 21 may be made of the same thermally conductive material, or they may be made of different materials with different characteristics regarding thermal conductivity. The thermally conductive elements 20, 21 are in operative connection with separate temperature regulating units of the IVD analyzer (not shown), when the sensor device 1' is positioned in the IVD analyzer. This enables supply or extraction of heat to or from the primary fluidic conduit 2 and/or the secondary fluidic conduit 3 and optionally the reference conduit 4 of the sensor device 1' via the thermally conductive elements 20, 21 independently. According to the embodiment shown in **FIG. 4****,** the operating temperature in the primary fluidic conduit 2 is regulated by supplying or extracting heat via the first thermally conductive element 20. The operating temperature in the secondary fluidic conduit 3 can be separately regulated by supplying or extracting heat via the second thermally conductive element 21. An insulation layer may be implemented between the two thermally conductive elements 20, 21 to prevent temperature exchange between them.

The primary fluidic conduit 2 and the secondary fluidic conduit 3 share a common fluidic inlet 5. Alternatively, the primary fluidic conduit 2 and the secondary fluidic conduit 3 may have separate fluidic inlets, analogue to the embodiment depicted in **FIG. 3****.** The reference conduit 4 has a separate fluidic inlet 6, through which the reference solution is provided into the sensor device 1' from a reservoir in the IVD analyzer (not shown). The primary fluidic conduit 2, the secondary fluidic conduit 3, and the reference conduit 4 converge and lead into a common fluidic outlet 7, through which fluids are transported out of the sensor device 1' and back into the fluid system of the IVD analyzer, where they can be wasted (not shown). The fluidic inlets 5, 6 and the fluidic outlet 7 represent the fluidic contact element to the IVD analyzer. The reference sensory element of an ion-selective electrode sensor 11B and of an enzyme-coupled sensor 12B and a conductivity sensor 15 are formed along the reference conduit 4, wherein the reference sensory elements 11B, 12B are in operative connection with at least a part of the sensory elements 11A, 12A, 13, 14 in the two fluidic conduits 2, 3.

In order to direct a fluid into the primary fluidic conduit 2 or the secondary fluidic conduit 3 or into both fluidic conduits 2, 3 simultaneously, the sensor device 1' comprises a switchable valve 9, which is illustrated as rotatable valve in **FIG. 4****.** It may however also be implemented as a flap or shutter-like element or a magnetically actionable element, etc. The valve 9 is in operative connection (not shown) with the IVD analyzer when the sensor device 1' is positioned in the IVD analyzer, e.g. by a suitable mechanical, conductive or magnetic mechanism. This enables the valve 9 to be switched to a desired position. For example, in **FIG. 4** the valve is switched to a position that enables a fluid to be directed into the primary fluidic conduit 2, while at the same time blocking access to the secondary fluidic conduit 3. However, the valve 9 can also be positioned to allow the provision of a fluid into the secondary fluidic conduit 3 while blocking access to the primary fluidic conduit 2, or to both the primary fluidic conduit 2 and the secondary fluidic conduit 3 simultaneously.

A plurality of sensory elements 11A, 11B, 12A, 12B, 13, 14, 15 are formed on the substrate 10 facing the primary fluidic conduit 2, the secondary fluidic conduit 3 and the reference conduit 4. In the embodiment depicted in **FIG. 4****,** the ion-selective electrode sensors 11A, 11B comprise working electrodes 11A, formed in the primary fluidic conduit 2, and a reference electrode 11B formed in the reference conduit 4, where the working electrodes 11A and the reference electrode 11B are in operative connection with each other. The enzyme-coupled sensors 12A, 12B comprise of working and counter electrodes 12A and a reference electrode 12B, where the same set of working and counter electrodes 12A are arranged in the primary fluidic conduit 2 and the secondary fluidic conduit 3, respectively. The blood gas sensors 13 are formed in the primary fluidic conduit 2, whereas the pH sensor 14 is formed in both the primary fluidic conduit 2 and secondary fluidic conduit 3. The enzyme-coupled sensors 12A, 12B can be used to detect metabolites in a sample, such as glucose, lactate, etc. The blood gas sensors 13 can be used to determine e.g. *P*O₂*, P*CO₂*,* and the pH sensor 14 is used to determine the pH level of a fluid. Further, conductivity sensors 15 are formed on the substrate 10 to measure the ability of a fluid to conduct an electrical current.

The sensory elements 12A, 14 formed in the secondary fluidic conduit 3 are at least in part the same sensory elements as in the primary fluidic conduit 2, i.e. they are of the same type and intended for measuring the same parameters. According to the embodiment depicted in FIG. 4, the sensory elements that are the same in the primary fluidic conduit 2 and in the secondary fluidic conduit 3 are sensory elements with higher susceptibility to deterioration than the other sensory elements in the primary fluidic conduit 2, e.g. enzyme-coupled sensors 12A for measuring glucose and lactate or a pH sensor 14.

In order to perform operation workflows (e.g. sample measurement, QC sample measurement, calibration, rinsing, cleaning, conditioning), a respective fluid is provided into the primary fluidic conduit 2. For example, a biological sample can be introduced into the primary fluidic conduit 2 where it comes in contact with the respective sensory elements 11A, 12A, 13, 14, 15. A sample measurement can then be performed in order to detect analytes of interest. With the setup as illustrated in **FIG. 4****,** the sensory elements with higher susceptibility to deterioration 12A, 14 formed in the primary fluidic conduit 2 repeatedly come in contact with potentially deteriorating fluids (e.g. reagent, QC sample, cleaning solution) and may therefore deteriorate faster than anticipated. However, if a sensory element with higher susceptibility to deterioration 12A, 14 falls out of specification, the IVD analyzer is controlled to switch to an extended operating mode. The extended operating mode comprises enabling the secondary fluidic conduit 3 to receive fluids. The switchable valve 9 is thus switched to a position that establishes fluidic connection between the fluidic inlet 5 and both the primary fluidic conduit 2 and the secondary fluidic conduit 3. The IVD analyzer then provides fluids to the primary fluidic conduit 2 and the secondary fluidic conduit 3 for subsequent operation workflows. The switchable valve 9 may however also be switched to a position that enables directing fluids into the secondary fluidic conduit 3 only, but not into the primary fluidic conduit 2. In another example, the switchable valve 9 may be switched to a position that enables a first sample being directed into the primary fluidic conduit 2 but not into the secondary fluidic conduit 3, followed by switching the switchable valve 9 to a position that enables a second sample being directed into the secondary fluidic conduit 3 but not into the primary fluidic conduit 2. This allows for measurements of two different samples at the same time or at least temporally overlapping, thereby increasing sample throughput. The embodiment of a sensor device 1' as depicted in **FIG. 4** and the method referenced in **FIG. 2** allow to increase the in-use time of the sensor device 1'.

The electrical contact elements 16 are connected to the respective sensory elements via electrically conducting pathways printed onto the substrate 10 and represent the electrical contact points between the sensor device 1' and the IVD analyzer.

According to a further embodiment not illustrated in this disclosure, the same set of sensors may be formed in both the primary fluidic conduit 2 and the secondary fluidic conduit 3. Sample measurements can be conducted in the primary fluidic conduit 2 until e.g. the first sensory element in the primary fluidic conduit 2 reaches the end of its in-use time. Sample influx may then be directed into the secondary fluidic conduit 3 while blocking access to the primary fluidic conduit 2. Any subsequent operation workflows can then be conducted in the secondary fluidic conduit 3 until e.g. the first sensory element in the secondary fluidic conduit 3 reaches the end of its in-use time. Only then would the sensor device 1' have to be replaced by the operator, thus increasing the in-use time of the sensor device 1'.

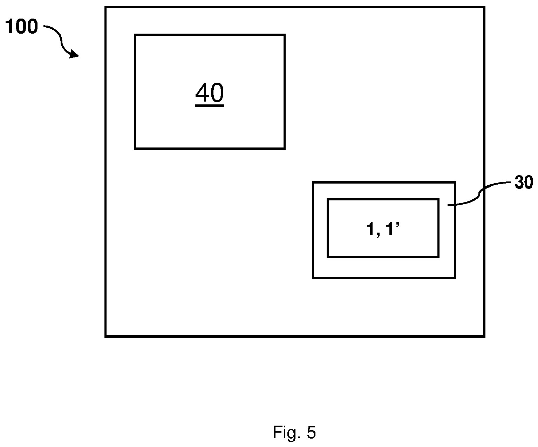

**FIG. 5** schematically shows an example of an IVD analyzer 100. The IVD analyzer 100 comprises a receptacle 30 for exchangeably receiving a sensor device 1, 1'. In another example, the sensor device 1, 1' is permanently installed in the IVD analyzer 100. When positioned in the receptacle 30 of the IVD analyzer 100, the sensor device 1, 1' is in operative connection with the IVD analyzer 100. The term "operative connection" thereby refers to e.g. thermal, electrical, fluidic, optical or mechanical connection. A connectivity check may be performed after installation of the sensor device 1, 1' in the IVD analyzer 100 in order to ensure a reliable and stable connection. With reference to certain embodiments, the receptacle 30 therefore comprises corresponding counterparts for connecting to the fluidic inlets and fluidic outlets, the electrical contact elements, the thermally conductive elements and the switchable valve of the sensor device 1, 1'. The IVD analyzer 100 further comprises a controller 40. The controller 40 is configured to control the IVD analyzer 100 to perform various operation workflows, e.g. sample measurement, QC sample measurement, calibration, rinsing, cleaning, convert electrical signal into analyte concentrations, displaying information to the operator, etc. In particular, the controller 40 is configured to control the IVD analyzer 100 to perform the methods as herein disclosed and referred to in **FIGS. 1** and 2. According to the embodiment depicted in **FIG. 5****,** the controller 40 is integrated into the IVD analyzer 100 and in communicative connection with other functional sub-units of the IVD analyzer 100. However, the controller 40 may alternatively be a separate logic entity in communication with the IVD analyzer 100, e.g. implemented on a computing device such as a desktop computer, a laptop, a smartphone, a tablet, PDA, etc.

In the preceding specification, devices and methods according to various embodiments are described in detail. The devices and methods may be embodied in many different forms and should not be construed as limited to the embodiments set forth and illustrated herein. It is therefore to be understood that the devices and methods are not to be limited to the specific embodiments disclosed and that modifications and other embodiments are intended to be included within the scope of the appended claims. Although specific terms are employed herein, they are used in a generic and descriptive sense only and not for purposes of limitation. Unless defined otherwise, all technical and scientific terms used herein have the same meaning as commonly understood by one skilled in the art to which the disclosure pertains. Although any methods and materials similar to or equivalent to those described herein can be used in the practice or testing of the methods, the preferred methods and materials are described herein.

Moreover, reference to an element by the indefinite article "a" or "an" does not exclude the possibility that more than one element is present, unless the context clearly requires that there be one and only one element. The indefinite article "a" or "an" thus usually means "at least one." Likewise, the terms "have," "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. For example, the expressions "A has B," "A comprises B" and "A includes B" may refer both to a situation in which, besides B, no other element is present in A (i.e. a situation in which A solely and exclusively consists of B) or to a situation in which, besides B, one or more further elements are present in A, such as element C, elements C and D, or even further elements.

Also, reference throughout the specification to "one embodiment", "an embodiment", "one example" or "an example", means that a particular feature, structure or characteristic described in connection with the embodiment or example is included in at least one embodiment. Thus, appearances of the phrases "in one embodiment", "in an embodiment", "one example" or "an example", in various places throughout this specification are not necessarily all referring to the same embodiment or example.

Furthermore, the particular features, structures, or characteristics may be combined in any suitable combinations and/or sub-combinations in one or more embodiments or examples, especially but not limited to the various ways of dividing the sensory elements among the different fluidic conduits.

## Claims

1. A method for operating a sensor device (1, 1') in an IVD analyzer (100), the sensor device comprising at least two fluidic conduits (2, 2', 3) for repeatedly receiving fluids, each fluidic conduit comprising at least one sensory element (11A, 11B, 12A, 12B, 13, 14, 15) arranged such as to come in contact with the fluid in the respective fluidic conduit (2, 2', 3),
- wherein the at least two fluidic conduits (2, 2', 3) comprise at least two primary fluidic conduits (2, 2') comprising different sensory elements (11A, 12A, 13, 14, 15) respectively, wherein the sensory elements (11A, 12A, 13, 14, 15) are separated in the respective primary fluidic conduits (2, 2') according to susceptibility to deterioration or measuring principle or operational conditions or degree of interference,
the method comprising enabling the at least two primary fluidic conduits (2, 2') to receive fluids and repeatedly providing fluids to the at least two primary fluidic conduits (2, 2'), or
- wherein the at least two fluidic conduits comprise at least one primary fluidic conduit (2) and at least one secondary fluidic conduit (3), the secondary fluidic conduit (3) comprising at least in part the same sensory elements (11A, 12A, 13, 14, 15) as the at least one primary fluidic conduit (2),
the method comprising
- operating the sensor device (1') in a primary operating mode, wherein the at least one primary fluidic conduit (2) is enabled to receive fluids,
- repeatedly providing fluids to the at least one primary fluidic conduit (2),
- in response to a predetermined trigger event, switching to an extended operating mode, wherein the at least one secondary sample conduit (3) is enabled to receive fluids, and repeatedly providing fluids to the at least one primary fluidic conduit (2) and/or to the at least one secondary fluidic conduit (3).

2. The method according to claim 1 further comprising regulating the operating temperature in each fluidic conduit (2, 2', 3) separately.

3. The method according to any of claims 1 or 2 wherein the predetermined trigger event comprises detecting that the performance of at least one sensory element (11A, 12A, 13, 14, 15) in the at least one primary fluidic conduit (2) is out of specification.

4. The method according to any of the preceding claims wherein the extended operating mode comprises a conditioning step, the conditioning step comprising
- providing a conditioning fluid to the at least one secondary fluidic conduit (3) for conditioning of the at least one secondary fluidic conduit (3).

5. The method according to claim 4 comprising regulating the operating temperature of the at least one secondary fluidic conduit (3) to be higher than the operating temperature of the at least one primary fluidic conduit (2) while performing the conditioning step.

6. A sensor device (1, 1') for an IVD analyzer (100), comprising
- at least two fluidic conduits (2, 2', 3) for repeatedly receiving fluids, each fluidic conduit comprising at least one sensory element (11A, 11B, 12A, 12B, 13, 14, 15) arranged such as to come in contact with a fluid in the respective fluidic conduit (2, 2', 3);
- wherein the at least two fluidic conduits (2, 2', 3) comprise at least two primary fluidic conduits (2, 2') comprising different sensory elements (11A, 12A, 13, 14, 15) respectively, wherein the sensory elements (11A, 12A, 13, 14, 15) are separated in the respective primary fluidic conduits (2, 2') according to susceptibility to deterioration or measuring principle or operational conditions or degree of interference, or
- wherein the at least two fluidic conduits (2, 2', 3) comprise at least one primary fluidic conduit (2) and at least one secondary fluidic conduit (3), the secondary fluidic conduit (3) comprising at least in part the same sensory elements (11A, 12A, 13, 14, 15) as the at least one primary fluidic conduit (2).

7. The sensor device (1, 1') according to claim 6 wherein the sensory elements (11A, 11B, 12A, 12B, 13, 14, 15) comprise any of an electrochemical sensor or a conductivity sensor or an optical sensor.

8. The sensor device (1, 1') according to claim 7 wherein the sensory elements (11A, 11B, 12A, 12B, 13, 14) comprising an electrochemical sensor comprise any of an ion-selective electrode sensor (11A, 11B, 14) or an enzyme-coupled sensor (12A, 12B) or a blood gas sensor (13).

9. The sensor device (1, 1') according to any of claims 6 to 8 wherein the sensory elements (11A, 11B, 12A, 12B, 13, 14, 15), that are the same in the at least one primary fluidic conduit (2) and in the at least one secondary fluidic conduit (3), are sensory elements with higher susceptibility to deterioration than the other sensory elements in the primary fluidic conduit.

10. The sensor device (1, 1') according to claim 9 wherein the sensory elements (11A, 11B, 12A, 12B, 13, 14, 15) with higher susceptibility to deterioration comprise any of an enzyme-coupled sensor (12A, 12B) and/or an ion-selective electrode sensor (11A, 11B, 14).

11. The sensor device (1, 1') according to any of claims 6 to 10 comprising a reference conduit (4) for receiving a reference solution, the reference conduit (4) comprising at least one reference sensory element (11B, 12B, 15), the at least one reference sensory element (11B, 12B, 15) being arranged to come in contact with the reference solution in the reference conduit (4), the at least one reference sensory element (11B, 12B, 15) further being in operative connection with at least a part of the sensory elements (11A, 12A, 13, 14, 15) in the at least two fluidic conduits (2, 2', 3).

12. The sensor device (1, 1') according to any of claims 6 to 11 comprising a plurality of thermally conductive elements (20, 21) for regulating the operating temperature in each fluidic conduit (2, 2', 3) separately.

13. The sensor device (1, 1') according to any of the claims 6 to 12 comprising at least one common fluidic inlet (5) for the at least two fluidic conduits (2, 2', 3) and comprising a switchable valve (9) for directing fluids into any of the at least two fluidic conduits (2, 2', 3) separately or simultaneously.

14. The sensor device (1, 1') according to any of the claims 6 to 13 comprising at least one common fluidic outlet (7) in fluidic connection with each of the at least two fluidic conduits (2, 2', 3) and with the reference conduit (4).

15. An IVD analyzer (100) comprising
- a receptacle (30) for receiving a sensor device (1, 1') according to claim 6, wherein the sensor device (1, 1') is in operative connection with the IVD analyzer (100),
- a controller (40) configured to control the IVD analyzer (100)
- to enable the at least two primary fluidic conduits (2, 2') of the sensor device (1, 1') to receive fluids and
- to repeatedly provide fluids to the at least two primary fluidic conduits (2, 2') of the sensor device (1), or
- to operate the sensor device (1') in a primary operating mode, wherein the controller (40) controls the IVD analyzer (100) to enable the at least one primary fluidic conduit (2) to receive fluids, and controls the IVD analyzer (100) to provide fluids to the at least one primary fluidic conduit (2), and
- in response to a predetermined trigger event, to switch to an extended operating mode, wherein the controller (40) controls the IVD analyzer (100) to enable the at least one secondary sample conduit (3) to receive fluids, and controls the IVD analyzer (100) to provide fluids to the at least one primary fluidic conduit (2) and/or the at least one secondary fluidic conduit (3).
